**Europäisches Patentamt**

**European Patent Office**

**Office européen des brevets**

(19)

(11) Numéro de publication : **0 148 896 B1**

(12) **FASCICULE DE BREVET EUROPÉEN**

(45) Date de publication du fascicule du brevet :
23.03.88

(51) Int. Cl.⁴ : **A 61 F 5/01**

(21) Numéro de dépôt : **84902589.5**

(22) Date de dépôt : **02.07.84**

(86) Numéro de dépôt international :
**PCT/FR 84/00164**

(87) Numéro de publication internationale :
**WO/8500285 (31.01.85 Gazette 85/03)**

(54) **CHAUSSURE ORTHOPEDIQUE POUR NOURRISSONS.**

(30) Priorité : **04.07.83 FR 8311779**

(43) Date de publication de la demande :
**24.07.85 Bulletin 85/30**

(45) Mention de la délivrance du brevet :
**23.03.88 Bulletin 88/12**

(84) Etats contractants désignés :
**AT BE CH DE FR GB LI LU NL SE**

(56) Documents cités :
**US-A- 3 171 407**
**US-A- 3 240 516**

(73) Titulaire : **MARCK, Thierry**
**Le clos des Baz Quai Warrens**
**F-74700 Sallanches (FR)**

**DUMOUTIER, Gérard Michel**
**13, rue des Allobroges**
**F-74700 Sallanches (FR)**

(72) Inventeur : **MARCK, Thierry**
**Le clos des Baz Quai Warrens**
**F-74700 Sallanches (FR)**
Inventeur : **DUMOUTIER, Gérard Michel**
**13, rue des Allobroges**
**F-74700 Sallanches (FR)**

(74) Mandataire : **Hagry, François**
**52, avenue de la Gare**
**F-74100 Annemasse (FR)**

**Description**

L'invention est relative aux chaussures orthopédiques destinées à corriger les malformations et mal-positions des pieds des nourrissons dès les premiers jours de la vie.

On sait qu'une très grande proportion, estimée être de l'ordre de 5 à 10 %, des nouveau-nés sont atteints de malformations et malpositions diverses des pieds : pied talus, valgus, varus, adductus, talus valgus talus varus, varus équin, métatarsus varus, creux, convexe... On rencontre également souvent des séquelles de pieds bots après les premiers traitements correcteurs.

De nombreuses méthodes et techniques orthopédiques et thérapeutiques sont actuellement utilisées pour réduire ces difformités dès les premiers jours suivant la naissance. On a ainsi recours à des bandages adhésifs élastiques, des bandages dits de Finck, des plâtres, des attelles, des matériaux thermoformables, et en traitement de consolidation à divers types de chaussures orthopédiques comme les chaussures anti-adductus, les sandales dites américaines et bottillons anti-varus.

Ces traitements, quelle que soit la qualité des résultats qu'on peut en obtenir, présentent des inconvénients notables :
- actes médicaux répétés et variés dont le succès dépend en grande partie de la formation, de l'habitude et de l'habileté du praticien ;
- utilisation inévitable de matériaux et de techniques de mise en œuvre multiples pour le traitement d'un seul et unique type de difformité ;
- risque important de troubles trophiques, qui peuvent rester cachés trop longtemps et présenter ainsi un caractère irréversible, engendrés par l'utilisation des plâtres, matériaux thermoformables et bandages adhésifs élastiques ;
- les chaussures orthopédiques existantes, telles les sandales américaines, les botillons anti-varus et anti-adductus ne sont susceptibles de s'appliquer qu'à un type simple de malformation, alors que la malformation présente la plupart du temps un caractère de combinaison avec des déviations de degrés variables dans plusieurs directions. Ainsi, le bottillon anti-adducteur décrit dans le brevet FR-A-2 467 560 comporte deux parties séparées correspondant respectivement à l'arrière pied et à l'avant-pied, articulées autour d'un axe vertical commun, et ne permet d'agir que sur un pied adductus simple puisque les deux parties ne peuvent pivoter l'une par rapport à l'autre que dans un plan horizontal. Par ailleurs, c'est dans ce cas un ressort de rappel qui est censé exercer l'action correctrice et les deux parties arrière et avant ne peuvent donc être immobilisées positivement l'une par rapport à l'autre dans la position de correction désirée.

Dans la chaussure proposée par le brevet US-A-3 171 407, où, en plus de l'articulation autour d'un axe vertical que l'on retrouve sous une forme différente, on se préocuppe en outre de la correction autour de deux autres axes, censés correspondre à deux autres degrés de liberté, cette correction n'est théoriquement réalisable qu'en déformant selon besoin une pièce de liaison constituée de deux branches parallèles. Outre le fait que cette déformation imposée ne peut être faite que de façon empirique en appliquant un effort « au juger », donc sans la précision la plus élémentaire, les deux axes étant pratiquement indéfinissables géométriquement et mécaniquement, de même que l'amplitude réelle de la déformation, cette déformation est de type plastique, donc irréversible et la pièce déformée est difficilement utilisable plus d'une fois ou deux sans être gravement endommagée, ce qui doit nécessiter de nombreux remplacements imposant le démontage et le remontage de nombreuses pièces. Par ailleurs, le dispositif décrit est d'une complexité extrême, ce qui ne peut guère en rendre la réalisation et l'utilisation envisageables.

La présente invention vise à s'affranchir des insuffisances de l'état de la technique qui viennent d'être évoquées en proposant une chaussure orthopédique de mise en place rapide et permettant de corriger sans faire appel à d'autres matériaux les différents types de malformations que l'on peut rencontrer, simples ou combinés dans plusieurs directions.

L'invention, dont les caractéristiques techniques sont reprises dans les revendications, est exposée dans la description qui suit et pour l'intelligence de laquelle, on se référera aux dessins dont :
- la figure 1 représente en perspective et de façon générale, une chaussure à laquelle est appliquée l'invention,
- la figure 2 montre en plan deux demi-semelles correspondant à la chaussure de la figure 1,
- la figure 3 illustre en coupe partielle longitudinale un premier mode d'exécution de liaison entre les demi-semelles précédentes conformément à l'invention,
- la figure 4 montre en coupe longitudinale et en vue de dessous, un second mode d'exécution de l'invention, et
- la figure 5 illustre en A, B, C, D, E, F et G un certain nombre de malformations classiques dont l'invention permet la correction,
- les figures 6A, 6B et 7 représentent respectivement un pied bot en vue de derrière, une application de l'invention à sa correction, en vue de derrière également, et en vue de côté.

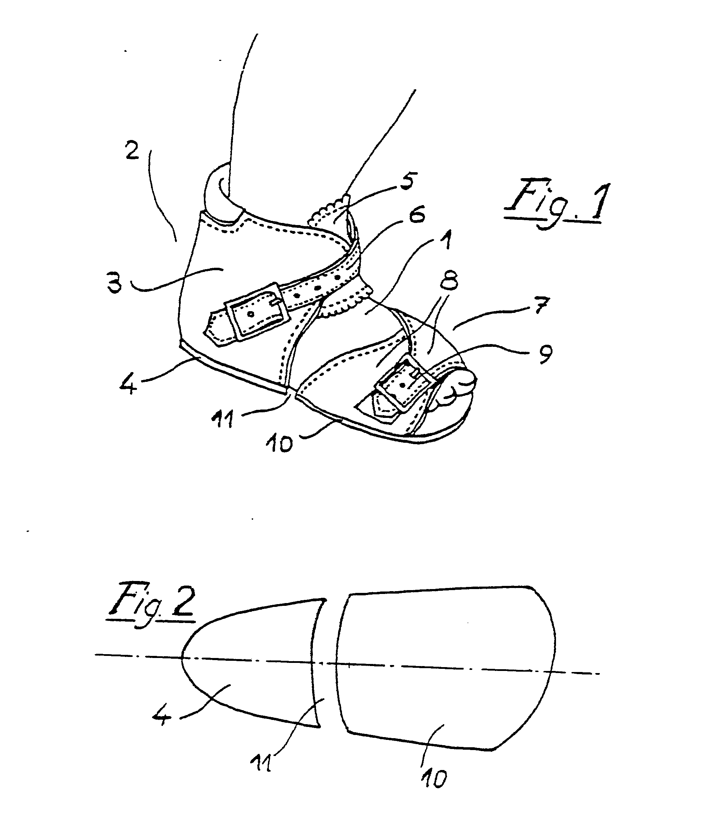

On voit à la figure 1 le pied 1 d'un nourrisson portant une chaussure orthopédique à laquelle peut être appliquée l'invention et dont les éléments essentiels non visibles sont décrits plus loin. La chaussure comporte deux parties séparées distinctes. Une partie postérieure 2, constituée de façon conventionnelle d'une tige rigide 3 montée sur une demi-semelle postérieure 4 avec une languette 5 et une bride de fermeture 6, sert au maintien ferme et confortable de l'arrière-pied. Une partie antérieure 7 servant au maintien de l'avant-pied comporte deux rabats latéraux 8 avec une bride de fermeture 9 montés sur une demi-semelle antérieure 10. Les deux demi-semelles 4, 10 constituent deux éléments distincts séparés par une solution de continuité ou jeu 11. Ce jeu 11 autorise un déplacement relatif au moins selon une certaine amplitude des deux parties 2, 7 sans chevauchement des demi-semelles 4, 10.

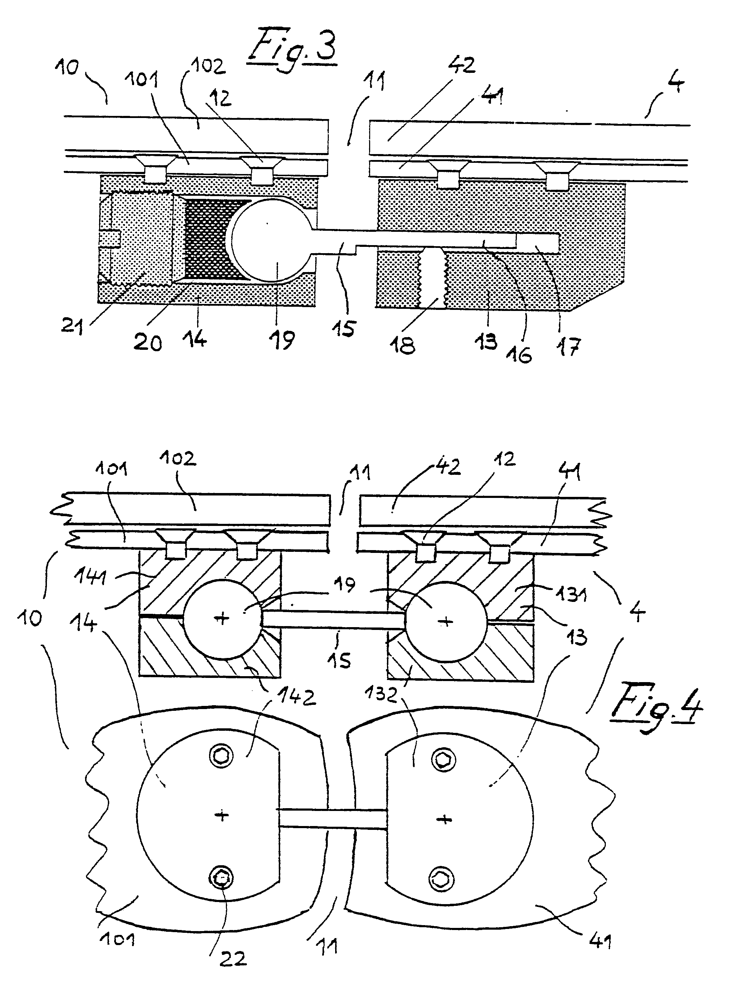

La figure 2 montre en plan une forme possible et la disposition relative des deux demi-semelles 4, 10 en position idéale pour un pied sans déviation par rapport au plan vertical moyen. Les demi-semelles 4, 10 comportent essentiellement une âme rigide 41, 101, de préférence en laiton ou aluminium, habillée sur sa face supérieure d'un revêtement, par exemple collé, adéquat pour le confort 42, 102, comme on le voit en coupe à la figure 3.

La méthode de correction d'une malformation du pied consiste à donner aux deux parties antérieure 7 et postérieure 2 une position relative telle qu'elle s'oppose à la malformation et à immobiliser ensuite les deux parties 2, 7 dans cette position. Cette correction doit se faire de façon graduelle et progressive dans l'espace et dans le temps pour rétablir une situation anatomique et physiologique normale, donc l'amplitude des déplacements relatifs des deux parties antérieure 7 et postérieure 2 doit pouvoir être variée à volonté par le praticien au moins dans une certaine gamme. Par ailleurs, comme il a été dit plus haut, on souhaite corriger, non seulement des malformations par rapport au plan vertical (pied adductus par exemple) mais aussi les déviations par rapport au plan horizontal et les malformations combinées, donc obliques par rapport à ces deux plans simultanément. Il est donc nécessaire que le déplacement relatif des deux parties antérieure 7 et postérieure 2 puisse se faire selon au moins deux degrés de liberté. La malformation pouvant en outre avoir aussi une composante de torsion autour d'un axe longitudinal par rapport au pied, il est nécessaire de disposer d'au moins un degré de liberté supplémentaire.

Dans le mode d'exécution représenté en coupe partielle longitudinale à la figure 3, chaque demi-semelle 4, 10 ou plutôt son âme 41, 101 est solidaire, par exemple au moyen de vis 12, d'un organe métallique, respectivement 13, 14 coopérant avec une pièce de liaison 15 en forme de tige unique pour assujettir entre elles les deux demi-semelles 4, 10. Ces organes 13, 14 peuvent être partiellement noyés dans les demi-semelles 4, 10 correspondantes. Dans le mode d'exécution illustré, l'extrémité 16 de la tige 15 coopérant avec la pièce 13 de la partie postérieure 2 de la chaussure, peut être déplacée dans un logement 17 en translation longitudinale et arrêtée en position, par exemple par une vis 18, de façon connue en soi.

A son autre extrémité, coopérant avec la pièce 14 associée à la partie antérieure 7 de la chaussure, la tige ou pièce de liaison 15 présente une partie sphérique 19 coopérant avec une cuvette sphérique correspondante formée en partie dans la pièce 14 et en partie dans un patin 20 qui peut venir serrer plus ou moins fortement la sphère 19 par un dispositif à vis 21 longitudinal ménagé dans la pièce 14. On a donc une rotule permettant au moins selon une certaine amplitude, tout déplacement en rotation de la demi-semelle 10 autour du centre de la sphère 19, déplacement s'analysant en ses trois composantes de rotation autour, respectivement, de l'axe vertical, de l'axe horizontal normal à la tige 15, et de l'axe de la tige 15. Cette demi-semelle 10 jouit donc de trois degrés de liberté par rapport à la demi-semelle 4. La vis 21 permet de freiner ces déplacements pour favoriser un positionnement correct par le praticien et de bloquer ensuite l'ensemble dans la position de réglage obtenue, sans qu'il y ait jamais déformation de la tige 15.

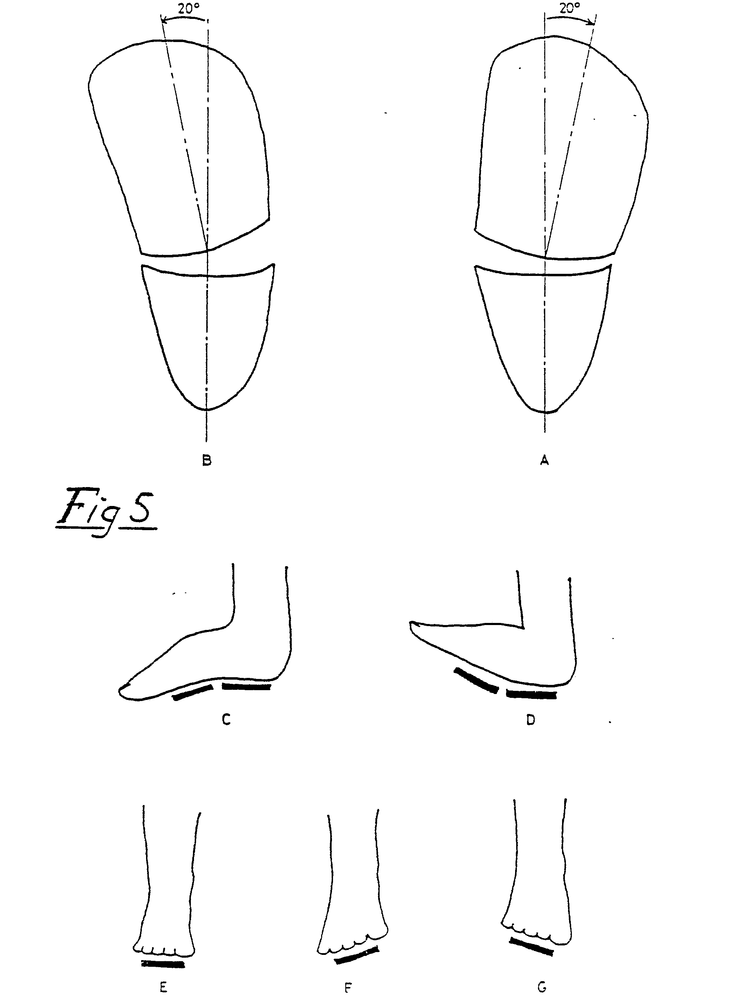

Dans un autre mode de réalisation représenté à la figure 4, la tige de liaison 15 présente à chaque extrémité une partie sphérique 19 coopérant avec une cuvette sphérique formée respectivement dans chaque pièce métallique 13 et 14. La solution de freinage et blocage par patin d'axe longitudinal précédente pourrait s'appliquer ici, mais, comme représenté, il semble plus avantageux de définir les cuvettes sphériques par deux demi-coquilles respectivement 131, 132 et 141, 142, la première 131, 141 solidaire de l'âme 41, 101 de la demi-semelle 4, 10 correspondante de la même manière que précédemment, la seconde 132, 142, venant se fixer sur la première au moyen d'au moins une vis 22 dont l'intensité du serrage, en appuyant les coquilles 131, 132 et 141, 142 sur les sphères 19 va permettre de freiner les déplacements relatifs de chaque demi-semelle autour du centre de la sphère 19 concernée, et de bloquer l'ensemble dans la position désirée. Cette solution à trois degrés de liberté pour chaque extrémité de la tige 15 donne une plus grande souplesse dans la recherche de la position de correction la mieux adaptée au cas d'espèce, et toujours sans déformation de la tige 15.

Dans le premier mode d'exécution décrit, il est possible avec une même tige 15 d'ajuster le jeu 11 pour qu'il corresponde à la pointure concernée dans le cas d'espèce. Dans le second mode, par contre, cet ajustement pourra se faire au moyen de tiges 15, de longueurs différentes, cette pièce étant facilement interchangeable.

D'autres solutions mécaniquement équivalentes peuvent évidemment être adoptées pour l'obtention du même résultat. Un habillage convenable pourra venir masquer la plus grande partie du dispositif sous la chaussure, et même la totalité après réglage s'il est amovible.

A titre d'exemple illustratif, la figure 5 montre, pour un pied droit, un certain nombre de malformations classiques que le dispositif selon l'invention permet de corriger, l'angle de déviation pouvant, pour chaque direction, atteindre environ 20°. On a ainsi en A une ouverture du pied (en dehors), en B une fermeture du pied (en dedans), en C un abaissement de l'avant-pied, en D une élévation de l'avant-pied, en E, F et G un pied en position respectivement horizontale, inclinée à droite et inclinée à gauche. Les positions A et B peuvent s'effectuer en position E ou se cumuler avec C et D et/ou F et G, du fait des trois degrés de liberté dont on dispose. Ces différentes positions sont toujours relatives à un point fixe de référence.

Par ailleurs, pour des raisons d'ordre pratique lors de l'utilisation, il est possible de prévoir parmi les organes de l'articulation, un dispositif non directement accessible au praticien et en rendant les différentes parties inséparables en cas de manoeuvre maladroite lors d'un déblocage exagéré, ce qui évitera de les éparpiller. Toute solution connue de l'homme de métier peut être utilisée à cet effet.

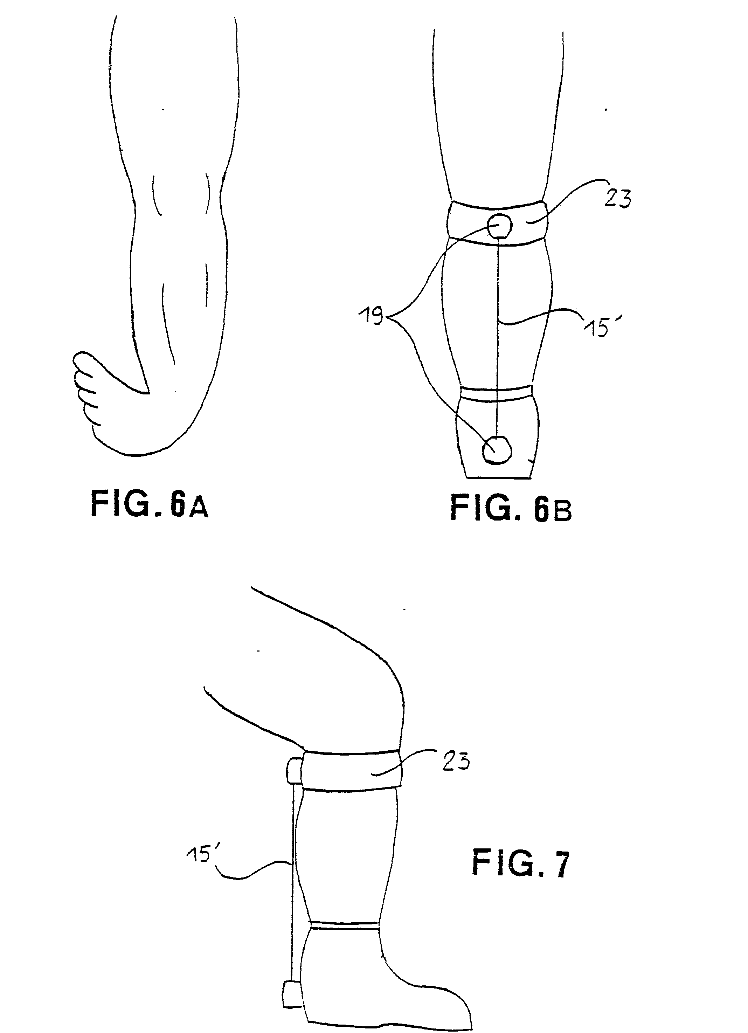

L'enseignement qui vient d'être donné peut en outre s'appliquer « mutatis mutandis » aux moyens de correction des malformations de l'articulation de la cheville, combinées ou non à des malformations du pied. Ainsi, les figures 6A, 6B et 7 illustrent très schématiquement le traitement d'un pied bot (figure 6A) par adjonction à la chaussure précédente d'une pièce de liaison 15' similaire de la pièce 15 (un peu plus longue) assujettie respectivement au talon de la chaussure et à un collier 23, par exemple de cuir, enserrant la jambe du sujet en-dessous et au-dessus du genou. Les extrémités 19 de la pièce de liaison 15', non représentées en détail, peuvent être identiques dans leur forme et leur assujettissement réglable au talon et au collier 23 à celles 16, 19 de la pièce 15 déjà décrite.

## Revendications

1. Chaussure orthopédique destinée à la correction des malformations du pied (1) chez les nourrissons, comprenant une partie postérieure rigide (2) pour recevoir et maintenir l'arrière-pied et une partie antérieure (7) pour recevoir et maintenir l'avant-pied, ces deux parties (2, 7) étant distinctes et assujetties l'une à l'autre par une pièce de liaison (15) permettant un déplacement articulé des parties (2, 7) l'une par rapport à l'autre selon au moins deux axes fictifs de pivotement, avec des moyens d'immobilisation en position sur au moins un de ces deux axes, caractérisée en ce que la pièce de liaison est constituée d'une tige unique (15) associée à des moyens (13, 14, 19, 20, 21) permettant sans déformation de la tige (15) un pivotement relatif des parties (2, 7) l'une par rapport à l'autre selon trois axes fictifs de pivotement et leur immobilisation en position.

2. Chaussure selon la revendication 1, caractérisée par le fait que chaque partie postérieure (2) et antérieure (7) comporte une demi-semelle (4, 10) et que ces deux demi-semelles (4, 10) sont séparées par un jeu (11) autorisant leur déplacement relatif sans chevauchement.

3. Chaussure selon la revendication 2, caractérisée par le fait que les moyens (13, 14, 19, 20, 21) associés à la pièce de liaison (15) pour permettre le déplacement relatif des deux parties (2, 7) sont portés au moins par l'une des deux demi-semelles (4, 10).

4. Chaussure selon l'une quelconque des revendications 1 à 3, caractérisée par le fait que la tige (15) porte au moins à une de ses extrémités, une partie sphérique (19) coopérant avec une cuvette sphérique (14, 20) portée par la demi-semelle (4, 10) correspondante pour former une articulation à rotule constituant les moyens permettant le déplacement relatif des deux parties.

5. Chaussure selon la revendication 4, caractérisée par le fait que la cuvette sphérique (14, 20) est constituée par au moins deux coquilles complémentaires (14, 20) pouvant être serrées (21) l'une contre l'autre, le degré de serrage permettant de faire jouer l'articulation à rotule ou au contraire de l'immobiliser en position.

6. Chaussure selon la revendication 4 ou la revendication 5, caractérisée par le fait qu'une seule extrémité de la tige (15) porte une partie sphérique (19) et qu'à son autre extrémité (16) la tige est réglable en translation longitudinale et immobilisable (18) en position par rapport à la demi-semelle correspondante (4).

7. Chaussure selon la revendication 4 ou la revendication 5, caractérisée par le fait que la tige (15) porte une partie sphérique (19) à chacune de ses extrémités coopérant avec une cuvette sphérique correspondante (131, 132, 141, 142).

8. Chaussure selon l'une quelconque des revendications 1 à 7, caractérisée par le fait que la pièce de liaison (15) est interchangeable.

9. Chaussure selon l'une quelconque des revendications 1 à 8, caractérisée par le fait que la partie postérieure (2) est agencée de manière à pouvoir être assujettie à un collier (23), destiné à enserrer la jambe, par une pièce de liaison (15') associée à des moyens permettant un déplacement relatif de la partie postérieure (2) et du collier (23) l'un par rapport à l'autre.

10. Chaussure selon la revendication 9, caractérisée par le fait que les moyens permettant un déplacement relatif de la partie postérieure (2) et du collier (23) sont identiques à ceux (13, 14, 19, 20, 21) permettant un déplacement relatif des parties postérieure (2) et antérieure (7).

## Claims

1. An orthopedic shoe designed for correcting malformations on the foot (1) in infants, comprising a rigid posterior part (2) for receiving and holding the hindfoot and an anterior part (7) for receiving and holding the forefoot, these two parts (2, 7) being distinct, and secured to each other by a connecting piece (15) permitting an articulated displacement of the parts (2, 7) in relation to each other around at least two imaginary rotational axes, with means for their immobilization in position on at least one of these two axes, characterized in that the connecting piece is in the form of a single rod (15) associated with means (13, 14, 19, 20, 21) permitting a relative rotation of the parts (2, 7) in relation to each other around three imaginary rotational axes and their immobilization in position without any deformation of the rod (15).

2. A shoe according to claim 1, characterized in that each posterior (2) and anterior (7) part includes a half-sole (4, 10) and that these two half-soles (4, 10) are separated by a clearance (11) that allows their relative displacement without overlap.

3. A shoe according to claim 2, characterized in that the means (13, 14, 19, 20, 21) associated with the connecting part (15) to permit the relative displacement of the two parts (2, 7) are carried by at least one of the two half-soles (4, 10).

4. A shoe according to any of the claims 1 to 3, characterized in that the rod (15) bears, at least at one of its extremities, a spherical part (19) cooperating with a spherical cup (14, 20) carried by the corresponding half-sole (4, 10) to form a ball-and-socket joint constituting the means that permit the relative displacement of the two parts.

5. A shoe according to claim 4, characterized in that the spherical cup (14, 20) consists of at least two complementary shells (14, 20) that can be tightened (21) against each other, and the degree of tightening permits the ball-and-socket joint to work or, on the contrary, to be immobilized in position.

6. A shoe according to claim 4 or claim 5, characterized in that only one extremity of the rod (15) bears a spherical part (19) while at its other extremity (16) the rod is adjustable in a longitudinal movement of translation and can be immobilized (18) in position in relation to the corresponding half-sole (4).

7. A shoe according to claim 4 or claim 5, characterized in that the rod (15) bears a spherical part (19) at each of its extremities, cooperating with a corresponding spherical cup (131, 132, 141, 142).

8. A shoe according to any of the claims 1 to 7, characterized in that the connecting part (15) is interchangeable.

9. A shoe according to any of the claims 1 to 8, characterized by the fact that the posterior part (2) is designed in such a way that it can be secured to a collar (23), intended for enclosing the leg, through a connecting part (15') associated with means that permit a relative displacement of the posterior part (2) and the collar (23) with respect to each other.

10. A shoe according to claim 9, characterized in that the means that allow a relative displacement of the posterior part (2) and the collar (23) are identical to those (13, 14, 19, 20, 21) that allow a relative displacement of the posterior (2) and anterior (7) parts.

**Patentansprüche**

1. Orthopädischer Schuh zur Korrektur von Missbildungen des Fusses (1) bei Säuglingen, mit einem rückwärtigen steifen Teil (2) zur Aufnahme und Halterung der hinteren Fusspartie und einem vorderen Teil (7) zur Aufnahme und Halterung der vorderen Fusspartie, wobei diese zwei Teile (2, 7) in Abstand voneinander durch ein Verbindungsteil (15) miteinander verbunden sind, welches eine gelenkige relative Verstellung der Teile (2, 7) zueinander nach wenigstens zwei imaginären Schwenkachsen ermöglicht, mit Organen zur Blockierung der Stellung auf mindestens einer dieser zwei Achsen, dadurch gekennzeichnet, dass das Verbindungsteil aus einem einzelnen Schaft (15) besteht, der in Verbindung mit Organen (13, 4, 19, 20, 21) ohne Verformung des Schaftes (15) eine relative Schwenkung der Teile (2, 7) zueinander nach drei imaginären Schwenkachsen und eine Blockierung ihrer Stellung ermöglicht.

2. Schuh nach Anspruch 1, dadurch gekennzeichnet, dass das rückwärtige (2) und vordere Teil (7) jeweils eine Halbsohle (4, 10) aufweist, und dass diese zwei Halbsohlen (4, 10) durch einen Spielraum (11) voneinander getrennt sind, der ihre relative Verstellung ohne Überlappung gestattet.

3. Schuh nach Anspruch 2, dadurch gekennzeichnet, dass die zur jeweiligen Verstellung der zwei Teile (2, 7) mit dem Verbindungsteil (15) in Verbindung stehenden Organe (13, 14, 19, 20, 21) von mindestens einer der zwei Halbsohlen (4, 10) getragen werden.

4. Schuh nach einem der Ansprüche 1 bis 3, dadurch gekennzeichnet, dass der Schaft (15) an wenigstens einem seiner Aussenenden ein kugelförmiges Teil (19) aufweist. welches mit einer von der entsprechenden Halbsohle (4, 10) getragenen kugelförmigen Schale (14, 20) als Kugelgelenk zusammenwirkt und die Organe bildet, welche die relative Verstellung der zwei Teile ermöglichen.

5. Schuh nach Anspruch 4, dadurch gekennzeichnet, dass die kugelförmige Schale (14, 20) aus mindestens zwei zusätzlichen Schalen (14, 20) besteht, die gegeneinander angedrückt (2) werden können, wobei der Grad des ausgeübten Druckes einerseits das Spiel des Kugelgelenks, andererseits seine Raststellung bestimmt.

6. Schuh nach Anspruch 4 oder Anspruch 5, dadurch gekennzeichnet, dass nur eines der Aussenende des Schaftes (15) ein kugelförmiges Teil (19) aufweist, und dass der Schaft an seinem anderen Aussenende (16) in der Längsverschiebung jeweils zur entsprechenden Halbsohle (4) einstellbar und in einer Raststellung (18) blokkierbar ist.

7. Schuh nach Anspruch 4 oder 5, dadurch gekennzeichnet, dass der Schaft (15) an seinen Aussenenden jeweils ein kugelförmiges Teil (19) aufweist, das mit einer entsprechenden kugelförmigen Schale (131, 132, 141, 142) zusammenwirkt.

8. Schuh nach einem der Ansprüche 1 bis 7, dadurch gekennzeichnet, dass das Verbindungsteil (15) austauschbar ist.

9. Schuh nach einem der Ansprüche 1 bis 8, dadurch gekennzeichnet, dass das rückwärtige Teil (2) derart angeordnet ist, dass es mit einem Ringband (23) zur Umschnürung des Beins über ein Verbindungsteil (15') verbunden werden kann, welches mit Organen verbunden ist, die eine relative Verstellung des rückwärtigen Teils (2) und des Ringbands (23) zueinander ermöglichen.

10. Schuh nach Anspruch 9, dadurch gekennzeichnet, dass die Organe zur jeweiligen Verstellung des rückwärtigen Teils (2) und des Ringbandes (23) mit den Organen (13, 14, 19, 20, 21) zur jeweiligen Verstellung des rückwärtigen (2) und des vorderen Teils (7) identisch sind.

*Fig. 1*

*Fig. 2*

*Fig.3*

*Fig.4*

2

_Fig 5_

FIG. 6A

FIG. 6B

FIG. 7